# EUROPEAN PATENT SPECIFICATION

(11) **EP 1 009 299 B1**
(45) Date of publication and mention of the grant of the patent: **05.02.2003**
(21) Application number: 97939264.4
(22) Date of filing: 04.09.1997
(51) Int. Cl.: A61B 17/24, A61C 17/00

(54) **DEVICE FOR CLEANING WITHIN THE ORAL CAVITY**
REINIGUNGSVORRICHTUNG FÜR DIE MUNDHÖHLE
DISPOSITIF POUR NETTOYER LA CAVITE BUCCALE

(30) Priority: 05.09.1996 NO 963699; 18.06.1997 WO PCT/NO97/00155
(43) Date of publication of application: 21.06.2000
(73) Proprietor: One Drop Only Chemisch-pharmazeutische Vertriebs GmbH, 13627 Berlin (DE)
(72) Inventor: Molster, Olav, 6901 Floro (NO)
(74) Representative: Omsels, Hermann-Josef
(86) International application number: NO9700233
(87) International publication number: WO98009573

(56) References cited:
- GB-A- 773 038
- GB-A- 2 234 903
- GB-A- 2 260 905
- US-A- 697 336
- US-A- 1 891 864

## Description

The present invention relates to a device in an instrument for cleaning of the oral cavity as will be evident from the introduction of the following claim 1. Thus the invention relates to a cleaning instrument which comprises a handle element and a cleaning member connected to this comprising a scraping edge and a bristle portion.

With the present invention the aim is to alleviate the problem which many people have with bad breath.

The oral cavity (cavum oris) consists of teeth, cheeks, soft palate, tongue, mouth floor, transition folds and cheek mucous membranes. All these components can collect bacteria/-plaque, food residues and dead skin cells. With toothless people the roof of the mouth/the palate and floor of the mouth including the lower jaw (mandibula) can be a deposit for this collection of material. Several investigations have shown that thorough cleaning of the tongue and particularly its rear portions will reduce the afore-mentioned frequency of bad breath, that will increase the sense of taste and have the effect of refreshing the oral cavity. Thorough cleaning of the teeth is normal to-day in the population, but large geographical and social variations exist. Concerning other cleaning of the oral cavity then the western world is to-day little focussed on other than the brushing of teeth and interdental cleaning with tooth floss and tooth picks. The phenomenon of bad breathe can stem from the afore-mentioned conditions, but a series of research workers have published investigations where the main problem is the rear portions of the tongue. As regards persons without teeth collections of dead skin cells, fungi and bacteria will not only occur in the prosthesis itself, but also in cheeks and portions of the palate. It is also known that dead skin cells in the mucous embranes can influence the aroma of the air from exhalation.

A series of instruments are known for the removal of accumulations of odour-forming material on the tongue, and reference shall be made here to GB-patent specifications 2.027.347, 2,234,903, 2.260.905 and 2.265.831. These patent specifications show a U-shaped bow which comprises a scraping edge and two handles which the user grips on, after which he can scrape the tongue with the scraping edge of the bow. However this scraping does not give a sufficient cleaning of the tongue and there is no discussion either of cleaning portions of the oral cavity as is mentioned above. In addition a cleaning instrument is known from US patent specification 1.891.864 which comprises a combination of bristles and scraping edge. This instrument is however large and broad and long and it comprises up to six rows of bristles. It will therefore take a relatively large space in the oral cavity and there is the danger that the user gets vomiting feelings. The scraping edge which in the patent is shown by the reference number 4, is constituted by a separate metal plate, and it appears to consist of a sharp edge, which in itself can lead to the tongue being caused cutting injuries, or the like.

The US patent 697,336 describes a tongue-cleaner, which is U-shaped in contour and triangular in cross-section, providing with a smooth upper surface a knife-edge and having a lower surface o the body portion, which is flatten down. At the lower surface of the portion are bristles secured, which are arranged approximately centrally of the lower surface.

The British patent GB 773,038 refers to a tongue-scraper of metal, plastic or any other suitable material, comprising a rod bent to form a loop, by which the ends are joined to be united to a suitable handle. This leads to a reduced cleaning effect, because bristles or brushes are not part of the devices and there is only a mechanical cleaning by the flat surface of the rod. Small particles will not be removed by the device.

It is therefore an object of the present invention to produce a new instrument for cleaning of the oral cavity, and which remedies the drawbacks which are outlined above for the known cleaning instruments.

Devices in the instrument according to the present invention for cleaning within the oral cavity are characterised by the features which are evident from the characterising portion of the following patent claim 1. Preferred constructions are evident from the dependent claims.

According to the invention the device is used for the cleaning of the tongue, the mucous membranes, and if necessary the palate portion within the oral cavity. It is preferred to use the device together with a cleaning agent, such as a paste, gel or mouthrinse to be placed on the bristles.

Thus the cleaning member is formed of a substantially straight or slightly curved, if desired U-shaped bow, and a first side surface of the cleaning member is designed with one or two rows of upwardly projecting bristles and the second side surface is designed with a scraping edge.

Advantages and details concerning the instrument according to the present invention shall now be explained further with reference to the enclosed Figures, wherein:
Figs. 1 and 2 show respectively a perspective view and a plan view of a preferred construction of the instrument according to the present invention.
Fig. 3 shows a cross-section, partly in perspective, of the bristle-carrying portion of the instrument.
Fig. 3a shows a cross-section of an alternative design of the scraping edge of the bristle-carrying portion of the instrument.
Fig. 4 shows an alternative construction of a cleaning instrument, which do not fall under the definitions of the present invention.
Figs. 5 and 6 show preferred constructions of the instrument comprising a two-legged handle, which do not fall under the definitions of the present invention.
Figs. 7 - 15 show alternative and preferred constructions of the cleaning instrument according to the invention comprising a one-legged handle.
Fig. 16 shows an alternative and preferred construction of the head part of the instrument.
Fig. 17 shows a side view of the head part of the instrument according to Fig. 16.

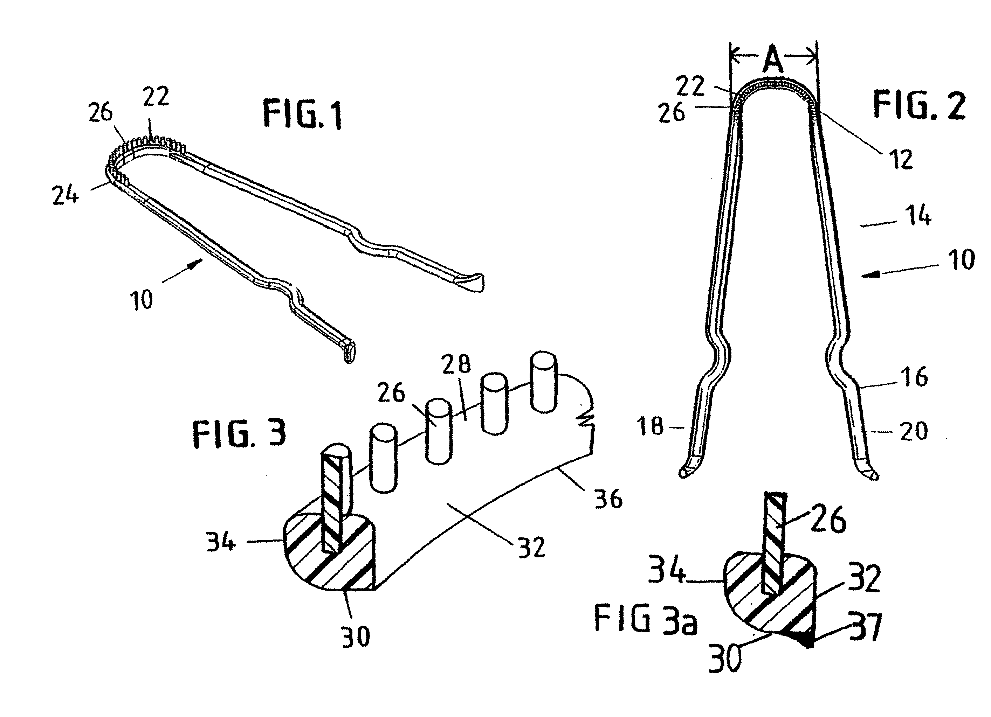

An instrument according to the present invention for cleaning different parts of the oral cavity is shown in Figures 1 and 2 by the reference numeral 10. The instrument comprises three main components, a front cleaning member 12, a middle section 14, and a rear handle member 16 (a shaft). The middle section 14 connects the cleaning member and the handle member 16 (the shaft).

The cleaning member 12 of the instrument 10 preferably forms a bow-shaped U-shaped bow profile, where each of the legs of the U shape is further connected, via the middle section 14, to the respective elongate handle parts 18,20. The handle parts (the legs) 18,20 are essentially designed so that the instrument can be gripped by the user's own hand with the thumb about the one leg 18 of the handle, while the other fingers are laid about the other handle 20.

The U-shaped bow-cleaning member 12 further comprises a first (bristle-carrying) side surface 22 and a second (scraping element-carrying) side surface 24. The first and second side surfaces 22 and 24 are substantially oppositely directed when looking in towards the belly part or back part of the bow (U-shape) according to Fig. 1, and form planes which are substantially parallel to the directions of the handles (the shafts) 18,20. The row of bristles 26 can be imbedded in the side surface, if desired be pressed into holes which are punched out in the side surface of the bow member.

The cross-section for the rod/bow can be square or rectangular with rounded corners, or can be circular, oval or the rod can have a composite cross-section of these forms.

With reference to Fig. 3, the bow member comprises the first (bristle-carrying) side surface 28, and the second (scraper element-carrying) side surface 30, these side surfaces being substantially oppositely directed. The wall-surface (belly side) 32 which faces inside the U-shape of the bow, and the wall surface 34 (back side) facing out of the U, connect respectively the two side surfaces 28 and 30. The side surfaces 28 and 30 form generally a right angle with the belly and back sides, but the elongate edge between the surfaces is rounded. The elongate edge which is defined between/by the belly surface 32 and the second side surface 30, form scraping edge 36 of the instrument. The two scraping edge-forming surfaces, namely the belly surface 32 and the second surface 30 can besides form dissimilar angles with each other, and the longitudinal edge 36 which is formed between them, will then have a varying sharpness dependent upon what the angle is between the surfaces 30 and 32. Obviously the sharpness is also regulated by the rounding of the edge 36. The edge 36 must be so much rounded that the user does not cut himself in the tongue, in the skin or the mucous membranes when he draws in normal fashion the instrument over the tongue in order to perform the scraping. Fig. 3 shows an alternative and preferred cross-sectional profile of the rod which forms the cleaning member 12. The rear side 34 and the second side surface 30 form together an arcuate shape, and preferably an approximately circular cross-section.

According to another preferred embodiment of the device of the invention, which appears on figure 3a, the scraping edge is defined by a ridge 37 formed at the second side surface 30 and an extension of the belly side 32, said ridge being elevated upwardly from said surface and extends along the arcuate shape of the bow. The ridge may be elevated from the surface in a range of approximately 1-3 millimetres. The ridge top edge is rounded in order not to effect cut injuries in the tongue in use.

The bristles 26 are installed as a row of bristles in the first side surface 28. The rows of bristles consists of a number of bundles of bristles which are each imbedded or pressed down in holes in the bow. The bristles 26 can be up to 8 mm. (millimetres) high, but preferably have a height of 3 mm., and can consist of bristles of dissimilar hardness. In the Figs. 1 - 3 a cleaning instrument is shown having one row of bristles, but two rows of bundles of bristles can also be used.

The distance or breadth between the outer edges of the front cleaning member 12 (or head portion) is denoted A on figure 2. The instrument according to the invention can be made with dissimilar breadths A adapted for adults and children. For an adult person the cleaning member 12 is made with a breadth of about 2,5-4 cm. something which approximately corresponds to the breadth of the tongue at the root of the tongue with an adult person. For children a breadth A of 2-2,5 cm may be suitable. An universal instrument intended for both children and adults may have a breadth (A) of 2-3 cm.

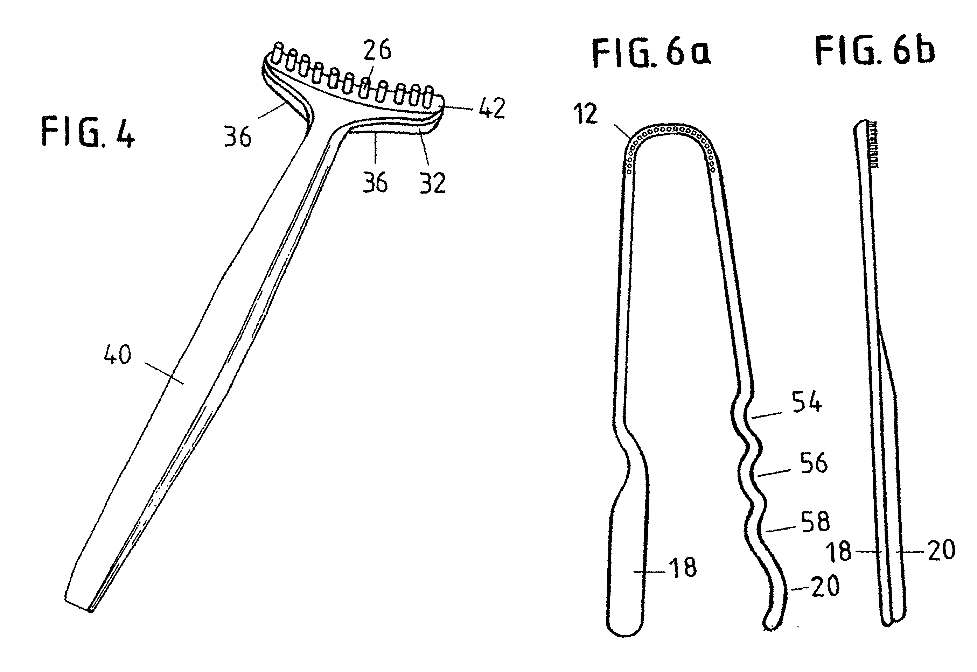

In Fig. 4 an alternative construction of a cleaning instrument, which do not fall under the definitions of the present invention. According to this alternative the instrument comprises only one handle 40, designed approximately as a conventional toothbrush. The one end of the handle 40 is connected to a transverse head portion 42 which then constitutes the cleaning member 12. The cleaning member has generally the same design with respect to the bristles 26, the scraping edge 36, the cross-sectional form, and the breadth as shown in Figs. 1 - 3 and discussed above. According to an alternative embodiment the head portion can be totally straight, as is indicated in Fig. 4. The handle is fastened midway between outer ends of the head portion 42.

According to an alternative construction both side surfaces 28 and 30 can be provided with a row of bristles. In order that the one edge shall function as a scraping edge the type of bristle installed ought then to be of a somewhat harder type than the row of bristles 26 on the opposite edge. This means that the instrument according to the invention can be equipped with separate rows of bristles having different softnesses.

There shall now be outlined how the cleaning instrument according to the invention is to be used.

The instrument 10 is gripped by the hand of the user and is guided backwards to the back portions of the tongue with the bristles 26 facing towards the rear of the tongue. Some scrubbing movements are undertaken forwards and backwards in the longitudinal direction of the tongue in order to loosen the material from the surface of the tongue, and that which sits in depth. In order to achieve a satisfactory scrubbing the instrument must be pressed down against the tongue with a given force.

Thereafter the instrument is taken out of the mouth, and it is turned so that the scraping edge faces towards the surface of the tongue. Again the instrument is guided backwards to the back portions of the tongue. With a given pressure a scraping of the tongue is undertaken by drawing the utensil from the back portions of the tongue and forwards to the front portion (the tip of the tongue). This operation is repeated four or five times, and one will then be able to see that a series of slime/plaque/bacteria has collected in the front portions of the tongue or that it lies as a coating on the scraping edge. This material is rinsed out of the mouth with clean water from the tap.

Thereafter the instrument is guided inside the mouth again, and now with the working bristles against the cheek mucous membranes on the right and then on the left side. The working edge is drawn thereafter 3 - 4 times from the back portions at the wisdom tooth region towards the corner of the mouth. This operation is repeated correspondingly on the opposite side.

During the scrubbing it is preferred that a cleaning agent is used. This can be placed on the tongue before the scrubbing with the bristles starts. Alternatively the cleaning agent can be placed on the row of bristles whereby it is scrubbed out over of the surface of the tongue. The cleaning agent can comprise a paste, gel, powder or have another suitable consistency. A paste can for example comprise conventional toothpaste from a tube and which is squeezed out and placed as a stripe up on or down in the row of bristles. Then it is ensured that the paste is spread satisfactorily out over the tongue.

With persons who do not have teeth one is also able to utilise the inventive instrument to clean the palate portion. The bristles are guided backwards to the transition between the hard and the soft palate, and one brushes forwards and to the side with small scrubbing movements.

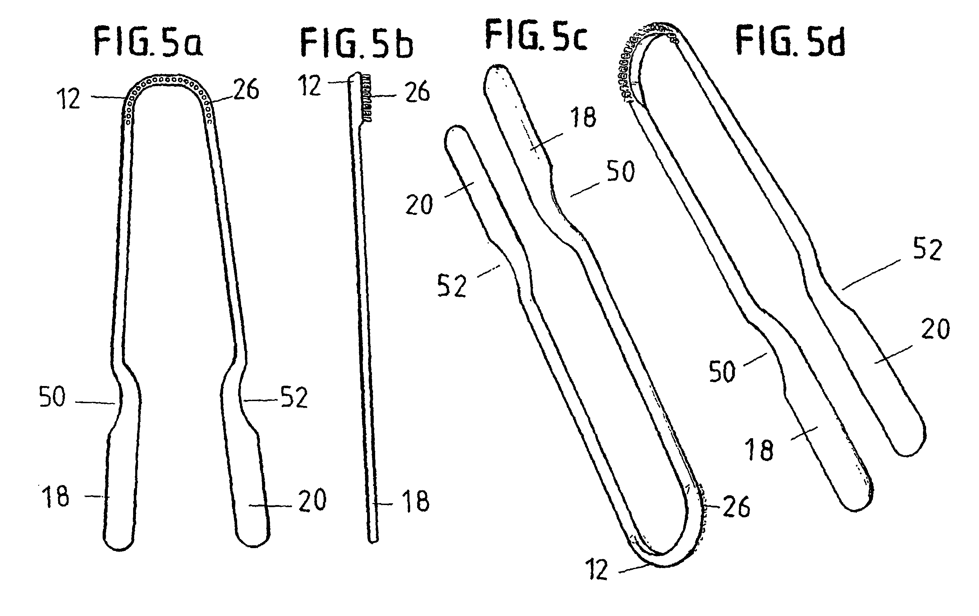

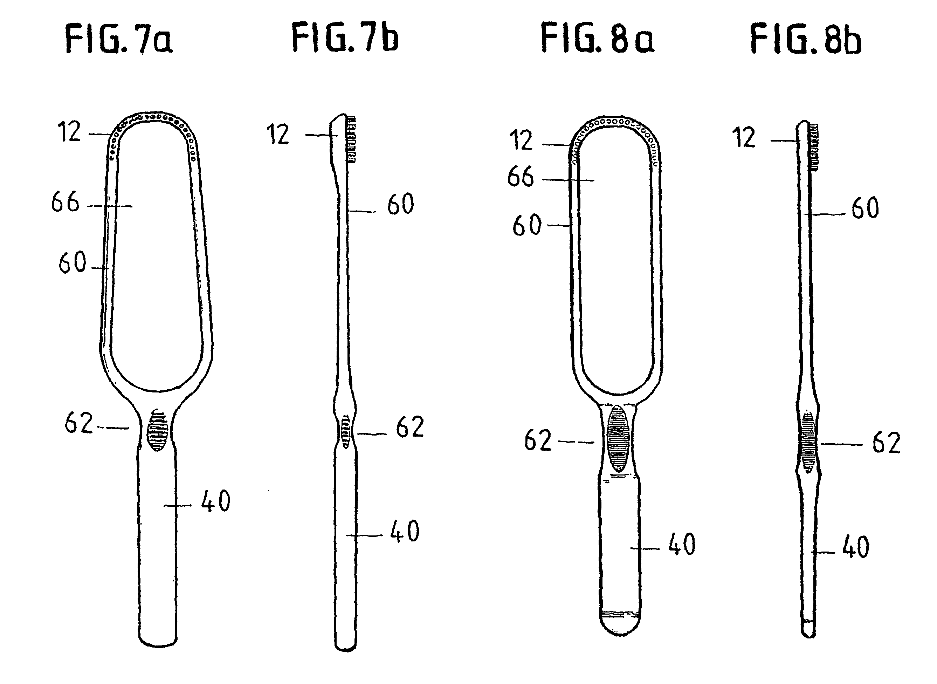

The Figures 5a-b, 5c-d, and 6 show three constructions where the cleaning head portion is roughly the same as discussed previously in connection with the Figures 1 - 2, while the difference lies mainly in the design of the handle member 16.

Figure 5a-b show a variant of an instrument, which do not fall under the definitions of the present invention, in a plan view and a side view respectively, while figure 5c-d shows perspective views seen from above and below respectively of an instrument, which do not fall under the definitions of the present invention.

According to the construction of Fig. 5a-b and 5c-d the handle legs 18,20 are broader in the plane section than the middle piece 14 and the head member 12. Further the handle legs 18,20 comprise like designed and laterally (from each other) directed concavities 50-52, where a person places the thumb and the forefinger during use of the instrument. The Figures 5a-b and 5c-d respectively show two variants of each other, the differences in these lying in the head portion. In the construction according to Figure 5a-b the shorter front bristle-carrying portion is approximately rectilinear, while in the construction according to Fig. 5c-d the head portion forms approximately a semi-circle.

According to Fig. 6 the right handle leg 20 is fashioned having a wave-shaped design with concavities 54,56,58 adapted for the placing of the user's fore, middle and ring fingers. This solution will give a still better control in the use of the instrument.

Fig. 7-12 show examples of cleaning instruments according to the invention comprising one legged handles, and where the middle piece 14 and the cleaning head 12 have generally the same form as the example according to Fig. 1 - 2. The middle piece 14 and the cleaning head form a bow-shaped oblong closed ring 60 one end of which comprises the head member 12, while the other end extends over into the handle 40 in a transition region 62.

The transition region 62 between the middle portion 60 and the handle member is in plan view designed with concave sides, so that there are formed one or more ring-shaped neck portion-forming grooves in the transition portion. The whole or parts of the groove can be designed with a rough surface. When the user grips the handle with his thumb and forefinger placed on each side of the neck portion, and against the rough surfaces, this gives a better grip. In the handle there can also be designed a number of small depressions, or a number of small elevations (warts) in the surface for improving the gripping characteristics of the handle.

Fig. 7 shows a construction where the leg of the middle piece 60 converges/tapers off somewhat forwards towards the head portion, and where in the neck portion there are formed a number of rough portions round the periphery of the neck portion.

Fig. 8 shows an embodiment where the leg of the middle piece 60 extends straight forwards towards the arcuate head portion 12, and where the tapered neck portion, which is formed with a number of rough portions around the periphery of the neck portion, has a somewhat larger longitudinal dimension than the example according to Fig. 7.

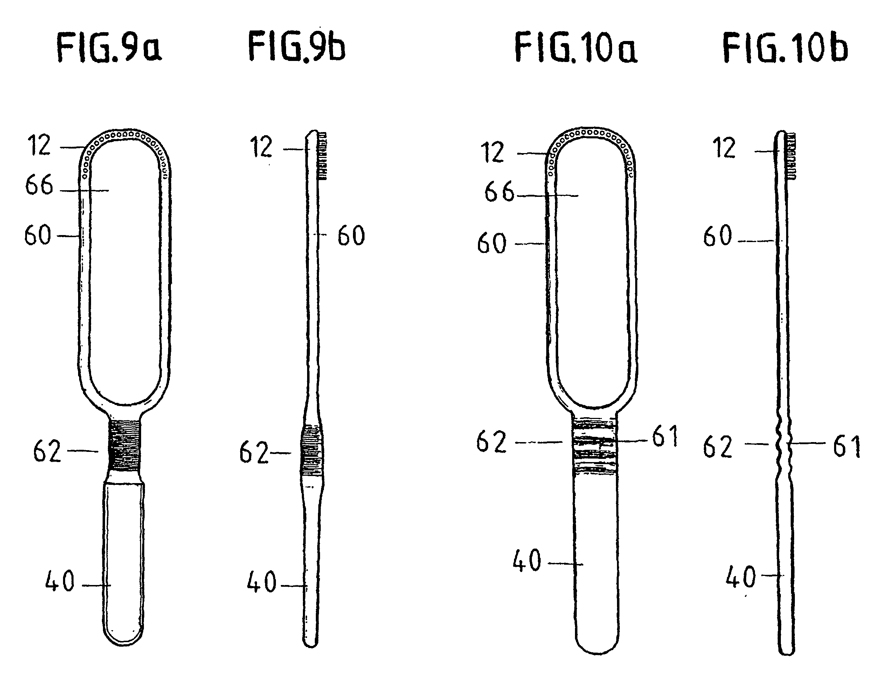

Fig. 9 also shows a construction where the leg of the middle piece 60 extends straight forwards towards the arcuate head portion 12, and where the whole of the tapered neck portion 62 is formed as a rough portion around the periphery of the neck portion, and has a length corresponding to the example according to Fig. 8.

The example according to Fig. 10 does not comprise any tapered neck portion in the transition between the handle member 40 and the middle portion, as the previous examples. This example comprises instead several coarse grooves 61 in the upper and under side, that is to say in the middle portion which defines that region where the thumb and forefinger are placed. In this construction also the leg of the middle piece 60 extends straight forwards towards the arcuate head portion 12.

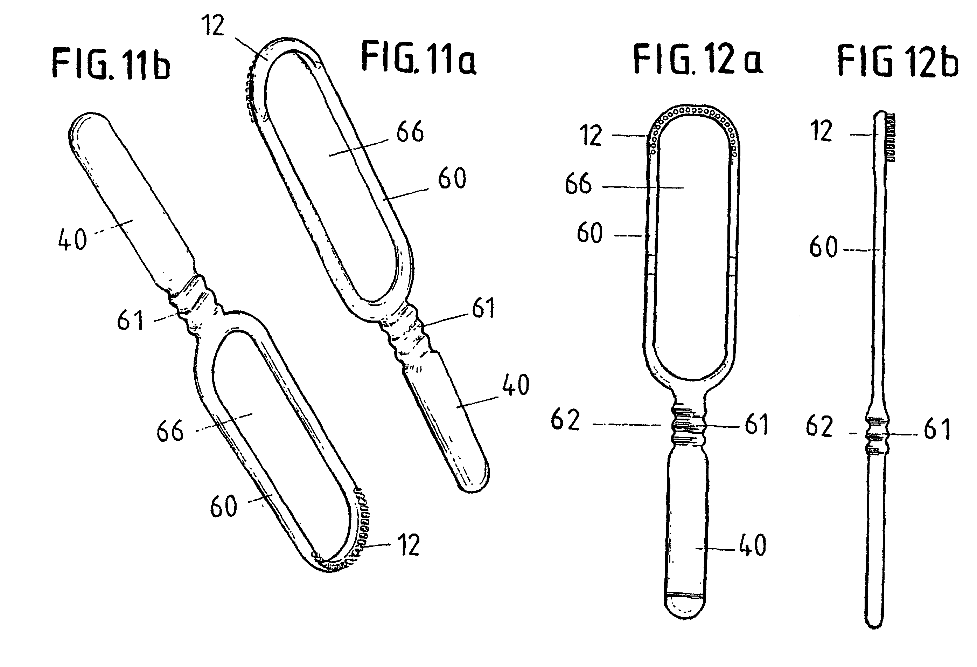

Fig. 11 shows two perspective views (obliquely from above and below respectively) of a variant of the cleaning instrument according to Fig. 10, the grooves 61 extending here around the whole of this part of the shaft 40 or the handle member 40. Further the shaft 40 is somewhat narrower than in the construction of Fig. 10.

Fig. 12 shows a similar construction of the cleaning instrument corresponding to Fig. 10, but where the marked tapered neck portion from the previously discussed solutions is retained. In this construction the shaft 40 is still somewhat narrower than in the construction according to Fig. 11.

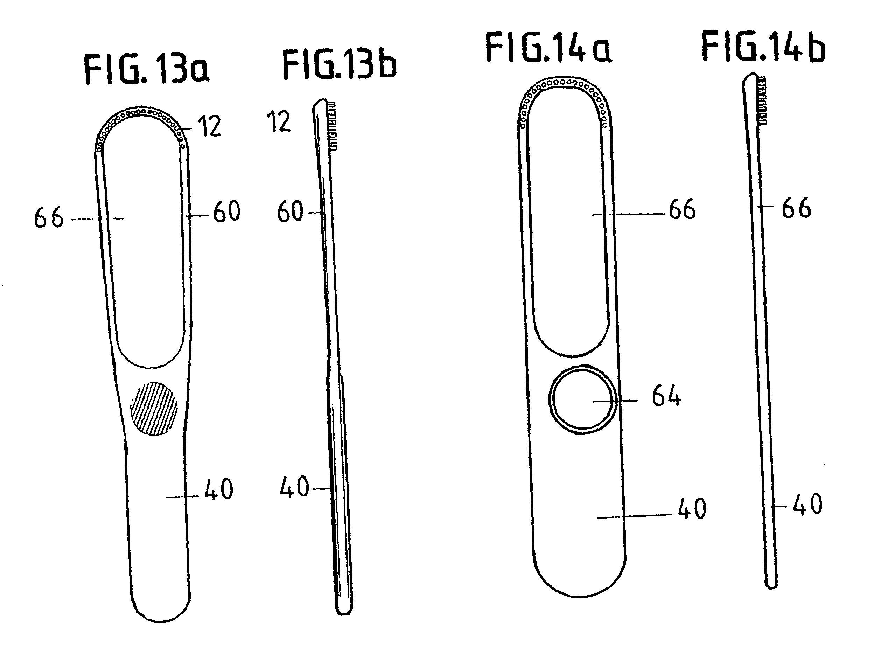

Fig. 13 shows a construction where the handle element extends with a gradually greater breadth towards the middle portion 60 which in turn becomes gradually broader (diverges) forwards towards the head portion. The arcuate head portion 12 with the row of bristles 26 is designed as in the former examples.

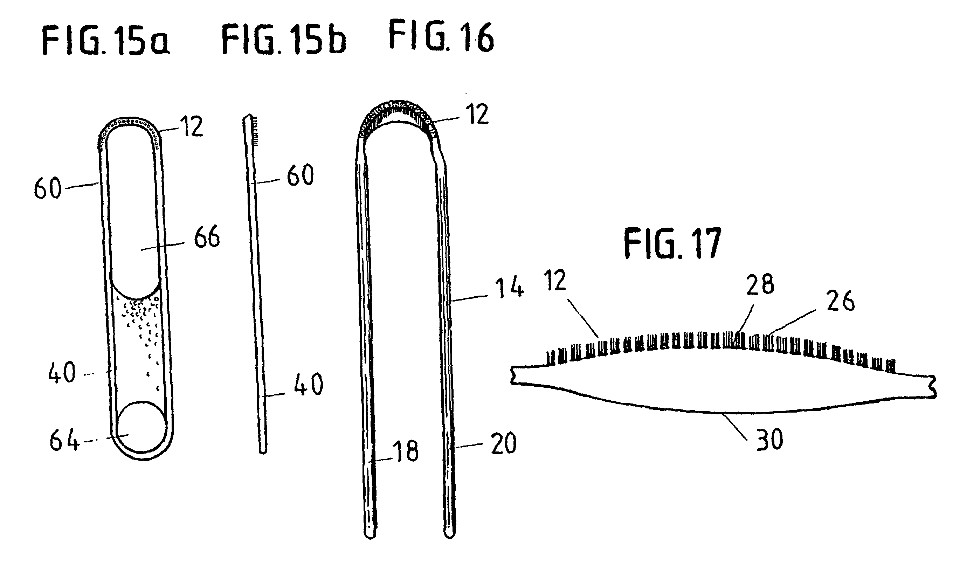

Fig. 14 and 15 show two constructions where the middle portion 60 extends directly over into the handle element 40, and where these are equally broad, that is to say that side edges of the instrument extend in parallel. According to the example of Fig. 14 the handle 40 comprises a through round hole 64 at the transition to the middle portion 60 and which the user partly grips into when he is to hold the instrument. In the example of Fig. 15 said hole 64 is placed at the back end of the handle 40.

Figs. 16 and 17 show an instrument with a two legged handle where respective side surfaces 28 and 30 form convex surfaces. The head portion thereby approximately acquires a cigar shape, as is especially evident from Fig. 17.

It is evident that all of the cleaning instruments with a one-legged handle, see the Figures 7 - 15, comprise a larger front open portion 66 which is surrounded by the front part of the handle, the middle portion-leg and the head member 12 projecting forwardly from there. On this point they resemble the construction with two legs. The large opening 66 functions so that when the user places the instrument backwards towards the root of the tongue, the front parts of the tongue can partially extend through the opening 66. If the opening 66 became "closed" by a sheet material, this would prevent the flexible use of the instrument according to the invention.

The cleaning instrument which is shown in the enclosed Figures and discussed in the foregoing can preferably be made of a suitable plastic material, that is to say plastic materials which are usually used in the manufacture of toothbrushes and similar instruments. However it is also possible to make the cleaning instrument of metals, but plastic materials are preferred.

With the described construction of the instrument according to the invention for cleaning of the regions within the oral cavity, the features can now be combined which apply to the loosening and thereafter the scraping of material which is collected in various parts of the oral cavity and which creates bad breath. Thus this constitutes a big improvement relative to the previously known instruments.

## Claims

1. Device in an instrument for cleaning within the oral cavity, especially the tongue, comprising a handle element (16) and connected to this a cleaning member (12), the cleaning member being formed by a substantially straight or slightly arcuate, or U-shaped, bow, thus defining a belly side (32) and a back side (34), and comprising a first side surface having two or more rows of upwardly projecting bristles (26) installed in the first side surface (28), and the cleaning member (12) comprising a scraping edge (36,37), **characterised in that** the first side surface (28) and a second side surface (30) of the cleaning member head portion (12) respectively, form convex surfaces resulting in an approximately cigar-shaped head portion and the second side surface (30) defining the scraping edge (36,37).

2. Device in accordance with claim 1, **characterised in that** the bristles (26) have a height of up to 8 mm (millimetres), preferably about 3 mm.

3. Device in accordance with claim 1 - 2, **characterised in that** bundles of bristles are utilised of dissimilar hardness.

4. Device in accordance with any of the preceding claims, **characterised in that** the first side surface (28) comprises regions without bristles (26).

5. Device in accordance with any of the preceding claims, **characterised in that** the scarping edge is defined by a ridge (37) formed at the second side surface (30) and of an extension of the belly side (32), said ridge (37) being elevated upwardly from the second side surface (30) and extends along the arcuate shape of the bow.

6. Device in accordance with claim 5, **characterised in that** the ridge top edge being rounded in order not to effect cut injuries in the tongue in use.

7. Device in accordance with claim 5-6, **characterised in that** the ridge is elevated from the second surface in a range of approximately 1-3 millimetres.

## Patentansprüche

1. Vorrichtung als Bestandteil eines Instruments für Reinigungszwecke innerhalb der Mundhöhle, insbesonders der Zunge, umfassend ein Handgriffelement (16), das mit einem Reiniger (12) verbunden ist, wobei der Reiniger als im wesentlichen gerade oder als etwas bogenförmige, oder als u-förmige Wölbung ausgebildet ist, und die Wölbung eine Vorderseite (32) und eine Rückseite (34) definiert, und umfassend eine erste Oberflächenseite, auf der zwei oder mehr Reihen von aufwärts hervorstehenden Borsten (26) in der ersten Oberflächenseite (28) angeordnet sind, sowie der Reiniger (12) einen Reinigungsschaber (36,37) aufweist, **dadurch gekennzeichnet, dass** die erste Oberflächenseite (28) und eine zweite Oberflächenseite (30) des Kopfteils des Reinigers (12) als konvexe Oberflächen ausgebildet sind, resultierend in einem ungefähr zigarrenförmigen Kopfteil und die zweite Oberflächenseite (30) somit den Reinigungsschaber (36,37) definiert.

2. Vorrichtung nach Anspruch 1, **dadurch gekennzeichnet, dass** die Borsten (26) eine Höhe von bis zu 8 mm (Millimetern), vorzugsweise 3 mm, haben.

3. Vorrichtung nach den Ansprüchen 1 - 2, **dadurch gekennzeichnet, dass** für die Borstenbündel ungleiche Borstenhärten verwendet werden.

4. Vorrichtung nach einem oder mehreren der vorgehenden Ansprüche, **dadurch gekennzeichnet, dass** die erste Oberflächenseite (28) Bereiche ohne Borsten (26) aufweist.

5. Vorrichtung nach einem oder mehreren der vorgehenden Ansprüche, **dadurch gekennzeichnet, dass** der Reinigungsschaber durch eine Kante (37) an der zweiten Oberflächenseite (30) definiert ist und als Verlängerung der Vorderseite (32) ausgebildet ist, wobei die Kante (37) sich aufwärts erhöhend auf der zweiten Oberflächenseite (30) befindet und sich entlang des bogenförmigen Abschnitts der Wölbung erstreckt.

6. Vorrichtung nach Anspruch 5, **dadurch gekennzeichnet, dass** der obere Rand der Kante abgerundet ist, um beim Gebrauch keine Schnittverletzungen an der Zunge zu verursachen.

7. Vorrichtung nach den Ansprüchen 5-6, **dadurch gekennzeichnet, dass** die Kante gegenüber der zweiten Oberflächenseite um eine Bereich von ungefähr 1-3 Millimeter überhöht ist.

## Revendications

1. Dispositif dans un instrument pour nettoyer l'intérieur de la cavité buccale, plus particulièrement la langue, comportant une poignée (16) et, raccordé à celle-ci, un élément nettoyant (12), l'élément nettoyant étant formé d'un arc essentiellement droit ou légèrement arqué, ou en forme de U, définissant ainsi une face inférieure (32) et une face supérieure (34) et comportant une première surface latérale portant deux ou plusieurs rangs de poils (26) dirigés vers le haut installés dans la première surface latérale (28) et l'élément nettoyant (12) comportant une arête raclante (36, 37), **caractérisé par le fait que** la première surface latérale (28) et une seconde surface latérale (30) de la tête de l'élément nettoyant (12) respectivement, forment des surfaces convexes ayant pour résultat une tête ayant à peu près la forme d'un cigare et la deuxième surface latérale (30) définissant l'arête raclante (36, 37).

2. Dispositif selon la revendication 1, **caractérisé par le fait que** les poils (26) ont une hauteur pouvant aller jusqu'à 8 mm (millimètres), de préférence de 3 mm.

3. Dispositif selon les revendications 1-2, **caractérisé par le fait que** les touffes de poils utilisées sont de dureté différente.

4. Dispositif selon n'importe laquelle des revendications précédentes, **caractérisé par le fait que** la première surface latérale (28) comporte des zones sans poils (26).

5. Dispositif selon n'importe laquelle des revendications précédentes, **caractérisé par le fait que** l'arête raclante est définie par une crête (37) formée au niveau de la deuxième surface latérale (30) et d'une extension de la face inférieure (32), ladite crête (37) s'élevant par rapport à la deuxième surface latérale (30) et s'étendant le long de la forme arquée de l'arc.

6. Dispositif selon la revendication 5, **caractérisé par le fait que** le bord supérieur de la crête est arrondi afin d'éviter des coupures de la langue pendant l'utilisation.

7. Dispositif selon les revendications 5-6, **caractérisé par le fait que** la crête s'élève d'environ 1 à 3 millimètres par rapport à la deuxième surface.
